# EUROPÄISCHE PATENTSCHRIFT

(11) **EP 2 024 318 B1**
(45) Veröffentlichungstag und Bekanntmachung des Hinweises auf die Patenterteilung: **12.09.2012**
(21) Anmeldenummer: 07724925.8
(22) Anmeldetag: 07.05.2007
(51) Int. Cl.: C07C 68/08, A23L 2/44, A23L 3/3463, A23L 3/3499, A23L 3/358

(54) **STABILISIERUNG VON DIKOHLENSÄUREDIESTERN DURCH FEINTEILIGE FESTSTOFFE**
STABILIZATION OF DICARBONIC DIESTERS BY FINELY DIVIDED SOLIDS
STABILISATION DE DIESTERS D'ACIDE DICARBOXYLIQUE PAR DES MATIERES SOLIDES FINEMENT PULVERISEES

(30) Priorität: 18.05.2006 DE 102006023243
(43) Veröffentlichungstag der Anmeldung: 18.02.2009
(73) Patentinhaber: LANXESS Deutschland GmbH, 51369 Leverkusen (DE)
(72) Erfinder: KAULEN, Johannes, 51519 Odenthal (DE); VOGL, Erasmus, 51373 Leverkusen (DE)
(86) Internationale Anmeldenummer: PCT/EP2007/004001
(87) Internationale Veröffentlichungsnummer: WO 2007/134710

(56) Entgegenhaltungen:
- EP-A1- 1 149 824
- WO-A-01/87096
- US-A- 2 910 400
- US-A- 3 186 906
- US-A- 3 792 158
- US-A- 3 979 524
- US-A- 5 738 888
- DATABASE WPI Week 197144 Derwent Publications Ltd., London, GB; AN 1971-70699S XP002445031 & JP 46 037810 B (SUMITOMO CHEM CO LTD) 8. November 1971 (1971-11-08) in der Anmeldung erwähnt
- DATABASE WPI Week 197306 Derwent Publications Ltd., London, GB; AN 1973-07971U XP002445176 & JP 48 004016 B (HODOGAYA CHEM CO LTD) 5. Februar 1973 (1973-02-05) in der Anmeldung erwähnt
- BIZRI, J. N. ET AL.: "CITRIC ACID AND ANTIMICROBIALS AFFECT MICROBIOLOGICAL STABILITY AND QUALITY OF TOMATO JUICE" JOURNAL OF FOOD SCIENCE, INSTITUTE OF FOOD TECHNOLOGISTS, CHICAGO, IL, US, Bd. 59, Nr. 1, 1994, Seiten 130-134, XP001079571 ISSN: 0022-1147

## Beschreibung

Die vorliegende Erfindung betrifft Mischungen enthaltend Dikohlensäurediester und feinteilige Feststoffe sowie die Verwendung dieser Mischungen zur Konservierung von technischen Materialien und Lebensmitteln.

Dikohlensäurediester werden unter anderem zur Konservierung von Lebensmitteln, als Bestandteile von antimikrobiellen Reagenzien, zur Deaktivierung von Enzymen in Fermentationsprozessen, oder zur Synthese von Feinchemikalien oder Polymeren verwendet. Dikohlensäurediester finden zudem z. B. als Katalysatoren zur Oxidation von Aminen oder zur Synthese, beispielsweise bei der Einführung von Schutzgruppen Verwendung.

Es ist bekannt, dass die Stabilität von Dikohlensäurediestern bei Raumtemperatur und insbesondere bei erhöhter Temperatur relativ gering sein kann. Insbesondere während der Aufreinigung, z. B. einer destillativen Reinigung, oder während einer längeren Lagerung kann es daher zur Zersetzung von Dikohlensäurediestern kommen. Diese Zersetzung kann die Qualität und Reinheit der Dikohlensäurediester verschlechtern. Zudem schreitet die Zersetzung allgemein umso schneller voran, je mehr Verunreinigungen enthalten sind. Eine hohe Reinheit sowie eine Stabilisierung von Dikohlensäurediestern sind daher sehr erstrebenswert.

Aus dem Stand der Technik sind bereits Verfahren zur Verbesserung der thermischen Stabilität von Dikohlensäurediestern bekannt. So wird z. B. vorgeschlagen, Dikohlensäuredialkylester durch den Zusatz von Metallsulfaten zu stabilisieren (vgl. JP-A 48-4016). Nachteilig ist hierbei jedoch, dass diese Metallsulfate mit den Dikohlensäuredialkylestern wenig bis schlecht mischbar sind.

Weiterhin ist bekannt, Dikohlensäuredialkylester durch den Zusatz von Borverbindungen zu stabilisieren (vgl. JP-A 46-37810). Nachteilig ist hierbei jedoch unter anderem ebenfalls die schlechte Mischbarkeit mit den Dikohlensäuredialkylestern.

Weiterhin sind Carbonylverbindungen sowie heteroanaloge Carbonylverbindungen vorgeschlagen worden, als die Lagerstabilität erhöhende Zusatzmittel für Lösungen von Dikohlensäuredialkylestern in gegenüber Dikohlensäuredialkylestern inerten Lösungsmitteln (vgl. DE-A 3231397). Allerdings sind stabilisierende Effekte nur mit prozentual relativ großen Mengen an Zusätzen zu erreichen.

Es bestand daher Bedarf an Stabilisatoren, die geeignet sind, Dikohlensäurediester noch wirkungsvoller gegen thermischen Zerfall zu schützen.

Überraschenderweise wurde nun gefunden, dass Dikohlensäurediester durch Zusatz bestimmter, (in Dikohlensäurediester unlöslicher) feinteiliger Feststoffe gegen thermische und/oder chemische Abbaureaktionen, wie sie z.B. bei der Lagerung oder der destillativen Reinigung auftreten können, sehr effizient stabilisiert werden können.

Gegenstand der vorliegenden Erfindung sind Mischungen enthaltend einen oder mehrere Dikohlensäurediester aus der Reihe Dimethyldicarbonat und Diethyldicarbonat und B₂O_{3,} mit einer durch Sieben ermittelten Korngröβe von ≤ 32 µm in einer Menge von allgemein 0.01 bis 100 000 ppm, bevorzugt von 0,1 bis 10 000 ppm, besonders bevorzugt von 0,1 bis 3000 ppm und ganz besonders bevorzugt von 0,1 bis 1000 ppm, bezogen auf den Dikohlensäurediester oder dessen Gemisch.

Teilchen geeigneter Abmessungen können durch Mahlen des Feststoffs bis zur gewünschten Korngröße und anschließendes fraktioniertes Sieben erhalten werden, wobei Sieben mit den entsprechenden Maschenweiten von ≤ 32 µm eingesetzt werden. Bei den Sieben handelt es sich z.B. um Analysensiebe der Fa. Retsch (nach ISO 565 / DIN 3310-1).

Die genannten allgemeinen, bevorzugten und besonders bevorzugten Feststoffe können mit den zum Zerkleinern üblicherweise zum Einsatz kommenden Geräten bis auf die gewünschte Korngröße gemahlen werden, beispielsweise in einer Kugelmühle oder einem Mörser.

Die feinteiligen Feststoffe können nach dem Zerkleinern direkt eingesetzt werden oder sie können geeignet vorsuspendiert werden. Zur Suspendierung eignen sich beispielsweise Dikohlensäurediester, Alkohole oder Wasser.

Die feinteiligen Feststoffe können auch auf Oberflächen immobilisiert zum Einsatz kommen. Als Matrices eignen sich dafür beispielsweise Aktivkohle, oder silikatische Trägermaterialien. Ebenso sind zur Immobilisierung organische Polymere wie beispielsweise Polyethylen, Polypropylen, Polyester, Polystyrol oder Polycarbonat als Matrices geeignet.

Die erfindungsgemäß stabilisierten Dikohlensäurediester zeichnen sich durch eine verbesserte Lagerstabilität aus. So können die so stabiliserten Dikohlensäurediester über mehrere Monate bei Raumtemperatur gelagert werden, ohne dass eine Zersetzung der Dikohlensäurediester zu beobachten ist.

Die erfindungsgemäßen Mischungen können über einen Zeitraum von mehreren Monaten gelagert werden, ohne dass es zu einer Zersetzung der darin enthaltenen Dikohlensäurediester kommt.

Die erfindungsgemäßen Mischungen eignen sich hervorragend zum Schutz und zur Konservierung von technischen Materialien und Lebensmitteln und insbesondere Getränken gegen Befall und/oder Zersetzung durch Mikroorganismen, wie beispielsweise Bakterien, Pilze oder Hefen. Ebenfalls Gegenstand der vorliegenden Erfindung ist die Verwendung der erfindungsgemäßen Mischungen zum Schutz von technischen Materialien und zur Konservierung vonLebensmitteln und Getränken.

Die erfindungsgemäβ stabilisierten Dikohlensäurediester eignen sich zum Biespiel in hervorragender Weise als Kaltentkeimungsmittel für stille oder karbonisierte Getränke wie Soft-Drinks, Vitamin-Getränke, Fruchtsaftgetränke, Teegetränke, alkoholische oder entalkoholisierte Wein-Getränke, Fruchtschorlen oder manche Biere. Üblicherweise werden dazu die Dikohlensäurediester in Mengen zwischen 10 und 250 ppm zeitnah zur Abfüllung den Getränken zugesetzt. Die Zumischung zu den Getränken erfolgt dabei mit speziellen Dosierpumpen. Die Dikohlensäurediester wirken dabei kontrollierend auf eine Reihe von Mikroorganismen wie fermentative Hefen, Schimmel oder fermentative Bakterien. Beispielhaft seien hier etwa genannt Saccharomyces cervisiae, Mycoderma, Brettanomyces spp, Lactobacillus brevis, Lactobacillus buchneri und viele andere.

Die erfindungsgemäß stabilisierten Dikohlensäurediester eignen sich weiterhin auch zum Schutz von technischen Materialien gegen Befall und Zerstörung durch unerwünschte Mikroorganismen.

Unter technischen Materialien sind im vorliegenden Zusammenhang nichtlebende Materialien zu verstehen, die für die Verwendung in der Technik zubereitet worden sind. Beispielsweise handelt es sich bei den technischen Materialien um Klebstoffe, Leime, Papier und Karton, Textilien, Leder, Holz, Holzwerkstoffe, Holzverbundstoffe, Anstrichmittel und Kunststoffartikel, Kühlschmierstoffe und andere Materialien, die von Mikroorganismen befallen oder zersetzt werden können. Weiterhin sind unter technischen Materialien im Rahmen der vorliegenden Erfindung auch Teile von Produktionsanlagen, beispielsweise Kühlwasserkreisläufe, zu verstehen, die durch Vermehrung von Mikroorganismen beeinträchtigt werden können. Bevorzugt zu schützende technische Materialien sind Klebstoffe, Leime, Papiere und Kartone, Leder, Holz, Holzwerkstoffe, Holzverbundstoffe, Anstrichmittel, Kunststoffartikel, Kühlschmiermittel und Wärmeübertragungsflüssigkeiten.

Insbesondere eignen sich die erfindungsgemäß stabilisierten Dikohlensäurediester der allgemeinen Formel (I) zum Schutz von Holz, Holzwerkstoffen, Holzverbundstoffen, Kunststoffen, Kühlschmiermitteln, wässrigen und oder lösemittelhaltigen organischen oder anorganischen Dispersionen und Beschichtungssystemen wie Anstrichfarben, Lacken oder Putzen vor dem Befall durch Mikroorganismen.

Als Mikroorganismen, die einen Abbau oder eine Veränderung der technischen Materialien genannt. Vorzugsweise wirken die erfindungsgemäß stabilisierten Dikohlensäurediester gegen Hefen, Bakterien und Pilze.

Es seien beispielsweise Mikroorganismen der folgenden Gattung genannt:
Acetobacter pasteurianus,
Aspergillus, wie Aspergillus niger,
Candida krusei
Chaetomium, wie Chaetomium globosum,
Escherichia, wie Escherichia coli,
Penicillium, wie Penicillium glaucum,
Pseudomonas, wie Pseudomonas aeruginosa,
Rhodotorula wie Rhodotorula rubra
Saccharomyces wie Saccharomyces cervisiae
Staphylococcus, wie Staphylococcus aureus.

Thermische Abbaureaktionen von Dikohlensäurediestern treten des weiteren insbesondere auch bei der Destillation von Dikohlensäurediestern auf, wie sie beispielsweise im Rahmen des Herstellungsverfahrens für Dikohlensäurediester durchgeführt wird. Durch die erfindungsgemäße Verwendung von feinteiligen Stabilisatoren gelingt es, Dikohlensäurediester mit geringeren Verlusten und in größerer Reinheit zu destillieren.

### Beispiele

Entsprechend den Angaben in den Tabellen 1 - 3 wurden jeweils definierte Mengen eines bestimmten, hochreinen Dikohlensäurediesters und die jeweils angegebenen Feststoffzusätze in einem 10 ml Rundkolben mit Magnetrührer eingewogen. Die jeweils verwendeten, genauen Mengen der Zusätze sind ebenfalls den Tabellen zu entnehmen.

Die Feststoffe wurden entweder direkt eingesetzt (grob) oder vorher fein mit einem Mörser gemahlen (fein). In diesen Experimenten wurde die erzielte Verringerung der Teilchengröße nicht näher bestimmt. Zur exakten Bestimmung der Teilchengrösse wurden dagegen Siebe mit definierten Maschengrößen (32 µm, 80 µm, 200 µm) verwendet. Damit konnten, die Siebung beginnend mit der kleinsten Maschenweite, Fraktionen von Korngrößen, wie in Tabelle 1 angegeben, aus dem vorher fein zerkleinerten Stabilisierungsmittel ausgesiebt werden.

Der Rundkolben wurde mit einem Septum fest verschlossen. In diesem Septum befand sich eine Öffnung, worin ein Teflonschlauch befestigt war, der in eine senkrecht gestellte, mit Silikonöl gefüllte. auf 0.1 ml kalibrierte 50 ml Bürette geleitet wurde. An der Skalierung der Bürette konnte die Menge des sich durch die Zersetzung des Dikohlensäurediesters entwickelnden Kohlendioxids abgelesen werden. Der Kolben wurde zügig in ein, wie in den Tabellen 1 - 3 für den jeweiligen Versuch angegeben, konstant temperiertes Ölbad (gerührt mit 500 rpm) abgesenkt. Die Eintauchtiefe des Kolbens betrug 2,0 cm.

Nach der jeweils angegebenen Zeit, in der Regel nach 1, 2, 5, 10 und 15 Minuten wurde das Gasvolumen abgelesen. Das Gasvolumen ist ein Maß für den Grad der Zersetzung des Dikohlensäurediesters. Es spiegelt damit umgekehrt den Grad der Stabilisierung durch die geprüften Zusätze wieder.

Die Ergebnisse sind den angehängten Tabellen zu entnehmen. Hochreiner Dikohlensäurediester setzte in der beobachteten Zeit wenig Kohlendioxid frei, doch schon in Kontakt mit geringen Mengen von Silicagel, wurde die Zersetzung drastisch beschleunigt. Je feinteiliger der Stabilisator desto höher die stabilisierende Wirkung.

**Tabelle 1**

| | | | | | | |
|---|---|---|---|---|---|---|
| **Diethyldicarbonat, 5000 ppm Zusatz an Feststoff-Stabilisator** | | | | | | |

| Temperatur [°C] | 130 | 130 | 130 | 130 | 130 | 130 |
|---|---|---|---|---|---|---|
| Diethyldicarbonat Menge [g] | 1 | 1 | 1 | 1 | 1 | 1 |
| Zusatz | ohne | Silikagel | Silikagel | Silikagel | Silikagel | Silikagel |
| Menge [mg] | | 10 | 10 | 10 | 10 | 10 |
| Zusatz Feststoff | ohne | ohne | B₂O₃ | B₂O₃ | B₂O₃ | B₂O₃ |
| Menge [mg] | | | 5 | 5 | 5 | 5 |
| Teikhengrösse [µm] | | | < 32µm | 32 - 80 µm | 80 - 200 µm | > 1 000 µm |

| **Gasentwicklung [**µ**m]** | | | | | | |
|---|---|---|---|---|---|---|
| Minuten 1 | **0,5** | **2,5** | **0,9** | **1,9** | **2,4** | **1,7** |
| Minuten 2 | **1,0** | **7,2** | **2,0** | **3,8** | **4,3** | **5,1** |
| Minuten 5 | **1,2** | **28,9** | **3,1** | **6,0** | **7,4** | **11,1** |
| Minuten 10 | **1,3** | **46,3** | **4,9** | **8,1** | **10,0** | **22,3** |
| Minuten 15 | **1,3** | **50,0** | **6,9** | **10,1** | **12,1** | **32,0** |

**Tabelle 2**

| | | | | | | |
|---|---|---|---|---|---|---|
| **Dimethyldicarbonat, 6670 ppm Zusatz Feststoff-Stabilisator** | | | | | | |

| Temperatur [°C] | 100 | 100 | 100 | 100 | 100 | 100 |
|---|---|---|---|---|---|---|
| Dimethyldicarbonat Menge [g] | 3 | 3 | 3 | 3 | 3 | 3 |
| Zusatz | ohne | Silikagel | Silikagel | Silikagel | Silikagel | Silikagel |
| Menge [mg] | | 10 | 10 | 10 | 10 | 10 |
| Zusatz Feststoff | ohne | ohne | Borsäure grob | Borsäure fein | B₂O₃ grob | B₂O₃ fein |
| Menge [mg] | | | 20 | 20 | 20 | 20 |

| **Gasentwicklung [ml]** | | | | | | |
|---|---|---|---|---|---|---|
| Minuten 1 | **0,1** | **1,0** | **0,9** | **0,4** | **0,8** | **0,7** |
| Minuten 2 | **0,2** | **3,4** | **3,3** | **1,8** | **2,8** | **2,2** |
| Minuten 5 | **0,6** | **20,3** | **7,7** | **3,9** | **7,3** | **4,9** |
| Minuten 10 | **0,8** | **46,1** | **10,0** | **5,1** | **12,7** | **6,4** |
| Minuten 15 | **1,3** | **50,0** | **10,9** | **6,1** | **15,8** | **7,2** |

**Tabelle 3**

| | | | | |
|---|---|---|---|---|
| **Dimethyldicarbonat, 1670 ppm Zusatz Feststoff-Stabilisator** | | | | |

| Temperatur [°C] | 100 | 100 | 100 | 100 |
|---|---|---|---|---|
| Dimethyldicarbonat Menge [g] | 3 | 3 | 3 | 3 |
| Zusatz Menge [mg] | ohne | Silikagel 10 | Silikagel 10 | Silikagel 10 |
| Zusatz Feststoff | ohne | ohne | B₂O₃ grob | B₂O₃ fein |
| Menge [mg] | | | 5 | 5 |

| **Gasentwicklung [ml]** | | | | |
|---|---|---|---|---|
| Minuten 1 | **0,1** | **1,0** | **0,4** | **0,4** |
| Minuten 2 | **0,2** | **3,4** | **2,1** | **1,8** |
| Minuten 5 | **0,6** | **20,3** | **9,8** | **6,1** |
| Minuten 10 | **0,8** | **46,1** | **22,6** | **12,6** |
| Minuten 15 | **1,3** | **50,0** | **32,9** | **16,6** |

## Patentansprüche

1. Mischungen enthaltend einen oder mehrere Dikohlensäuredialkylester aus der Reihe Dimethyldicarbonat und Diethyldicarbonat und B₂O₃, mit einer durch Sieben ermittelten Korngröβe von ≤ 32 µm in einer Menge von 0.01 bis 100 000 ppm, bezogen auf Dikohlensäuredialkylester oder deren Gemisch.

2. Verwendung einer Mischung gemäß Anspruche 1 zum Schutz und zur Konservierung von technischen Materialien, Lebensmitteln und Getränken.

## Claims

1. Mixtures comprising one or more dialkyl pyrocarbonates from the group consisting of dimethyl pyrocarbonate and diethyl pyrocarbonate and B₂O₃ having a particle size, determined by screening, of ≤ 32 µm in an amount of from 0.01 to 100 000 ppm, based on the dialkyl pyrocarbonate or its mixture.

2. Use of a mixture according to Claim 1 for protecting and preserving industrial materials, foodstuff and beverages.

## Revendications

1. Mélanges contenant un ou plusieurs esters dialkyliques d'acide dicarbonique choisis dans la série dicarbonate de diméthyle et dicarbonate de diéthyle et B₂O₃ ayant une taille de grain, déterminée par tamisage, de ≤ 32 µm en une quantité de 0,01 à 100 000 ppm, par rapport aux esters dialkyliques d'acide dicarbonique ou leur mélange.

2. Utilisation d'un mélange selon la revendication 1, pour la protection et pour la conservation de matériaux industriels, produits alimentaires et boissons.
